(19) Europäisches Patentamt
European Patent Office
Office européen des brevets

(11) Veröffentlichungsnummer: **0 272 213 B1**

(12) # EUROPÄISCHE PATENTSCHRIFT

(45) Veröffentlichungstag der Patentschrift: **10.07.91**

(51) Int. Cl.5: **C07C 45/30**, C07C 49/84, C07C 49/784, C07C 309/78

(21) Anmeldenummer: **87810733.3**

(22) Anmeldetag: **09.12.87**

(54) **Verfahren zur Oxidation sekundärer aromatischer Alkohole.**

(30) Priorität: **15.12.86 CH 4987/86**

(43) Veröffentlichungstag der Anmeldung:
**22.06.88 Patentblatt 88/25**

(45) Bekanntmachung des Hinweises auf die Patenterteilung:
**10.07.91 Patentblatt 91/28**

(84) Benannte Vertragsstaaten:
**DE ES FR GB IT**

(56) Entgegenhaltungen:
**DE-A- 2 902 541**
**DE-C- 912 092**

**METHODEN DER ORGANISCHEN CHEMIE, Band IV/1a, 4. Auflage,1981, Seite 325, Georg Thieme Verlag, Stuttgart, DE; HOUBEN-WEYL: "Dehydrierung zu Alkenen bzw. Arenen."**

**ADVANCED ORGANIC CHEMISTRY, 2. Auflage, 1984, Seite 452, McGraw-Hill International Book Company, London, GB; J. MARCH: "Aliphatic Nucleophilic Substitution"**

**PATENT ABSTRACTS OF JAPAN, Band 2, Nr. 152 (3551 C 78), 20.12.78; & JP-A-53 116 302**

**ORGANIC SULFUR COMPOUNDS, Band 1, 1961, Seiten 371-374, Pergamon Press, Oxford, GB; N. KHARASCH: "Trichloromethanesulfenyl Chloride and Trichloromethanesulfonyl Chloride"**

**SYNTHETIC METHODS OF ORGANIC CHEMISTRY, Band 13, 1959, Seite 4, S. Karger, Basel, CH; W. THEILHEIMER et al.: "Synthetische Methoden der Organischen Chemie - Jahrbuch"**

(73) Patentinhaber: **CIBA-GEIGY AG**
**Klybeckstrasse 141**
**CH-4002 Basel(CH)**

(72) Erfinder: **Rutsch, Werner, Dr.**
**Avenue Weck-Reynold 1**
**CH-1700 Fribourg(CH)**
Erfinder: **Slongo, Mario, Dr.**
**Sägetrainweg 553**
**CH-1712 Tafers(CH)**

**EP 0 272 213 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur Oxidation sekundärer aromatischer Alkohole, speziell solcher vom Type der Benzhydrole und Benzoine, zu den entsprechenden Ketonen. Aus den Benzhydrolen entstehen dabei die entsprechenden Benzophenone, aus den Benzoinen entstehen die entsprechenden Benzile. Als Oxidationsmittel verwendet man ein Sulfonsäurechlorid, das dabei zur entsprechenden Sulfinsäure reduziert wird. Das Verfahren dient also gleichzeitig zur Herstellung von Sulfinsäuren aus Sulfonsäurechloriden.

Zur Oxidation sekundärer aromatischer Alkohole zu den entsprechenden Ketonen sind bereits viele Verfahren vorgeschlagen worden. Einige dieser Verfahren sind mit Umweltproblemen belastet, wie z.B. die Oxidation mit konzentrierter Salpetersäure, bei der Stickoxide als umweltbelastende Nebenprodukte entstehen. Einige Verfahren arbeiten mit Schwermetallkatalysatoren wie z.B. Kupfer- oder Chromat-Katalysatoren, die in die Abwässer gelangen und damit ebenfalls die Umwelt belasten. Das vorliegende Verfahren arbeitet unter nicht-korrosiven Bedingungen, benötigt wenig thermische Energie, liefert keine umweltbelastenden Nebenprodukte und lässt sich zur gleichzeitigen Herstellung von Sulfinsäuren verwenden.

Das Verfahren ist anwendbar zur Herstellung aromatischer Ketone der Formel I

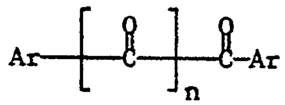

(I)

worin Ar ein aromatischer carbocyclischer Rest mit 6-14 C-Atomen oder aromatischer heterocyclischer Rest mit 4 - 14 C-Atomen ist, der unsubstituiert oder durch eine oder mehrere der Reste Halogen, $C_1$-$C_{14}$-Alkyl, $C_1$-$C_8$-Alkoxy, Phenyl oder Nitro substituiert sein kann, und n 0 oder 1 ist, unter gleichzeitiger Bildung einer Sulfinsäure der Formel R-S(O)-OH oder deren Alkalimetall- oder Erdalkalimetallsalz, worin R $C_1$-$C_{18}$-Alkyl, 10-Campheryl, Phenyl, Naphthyl, oder durch Halogen, $C_1$-$C_{14}$-Alkyl, $C_1$-$C_4$-Alkoxy, Phenoxy oder Phenyl substituiertes Phenyl oder Naphthyl ist, und ist dadurch gekennzeichnet, dass man einen sekundären Alkohol der Formel II

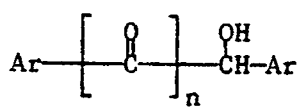

(II)

mit einem Sulfonsäurechlorid der Formel $R\text{-}SO_2\text{-}Cl$ in Gegenwart einer Base, ausgewählt aus Alkali- oder Erdalkalimetall-hydroxiden, -oxiden oder -alkoholaten, und in Gegenwart eines organischen Lösungsmittels bei einer Temperatur von 20° bis 150° C umsetzt, wobei pro Mol des Alkohols II mindestens 1 Mol $RSO_2Cl$ und mindestens 2 Aequivalente der Base verwendet werden.

Ar als carbocyclischer Rest in Formel I und II kann z.B. Phenyl, Naphthyl oder Phenanthryl, insbesondere Phenyl, sein. Substituierte carbocyclische Reste Ar sind z.B. Tolyl, Xylyl, 3-Ethylphenyl, 4-tert. Butylphenyl, 4-Octylphenyl, 4-Methoxyphenyl, 4-Ethoxyphenyl, 4-Isopropoxy-2-methylphenyl, 4-Chlorphenyl, 4-Bromphenyl, 2,4-Dichlorphenyl, 4-Fluorphenyl, 3-Nitrophenyl, 4-Biphenyl, 4-Nitro-2-naphthyl, 4-Chlor-1-naphthyl oder Hexadecylnaphthyl.

Ar als heterocyclischer Rest kann ein einkerniger oder mehrkerniger heterocyclischer Rest mit einem oder mehreren Heteroatomen sein. Die Heteroatome sind vorzugsweise O, N oder S. Beispiele für solche Reste sind Thienyl, Furyl, Pyrrolyl, Pyridyl, Chinolyl, Indolyl, Carbazolyl oder Thioxanthyl, insbesondere 2-Furyl, 2- oder 3-Pyridyl oder 3-Indolyl.

Ar als substituierter heterocyclischer Rest kann insbesondere ein alkylierter Heterorest sein, wie z.B. Methylfuryl, Methylpyridyl, Dimethylpyridyl, Ethylpyridyl oder Methylchinolyl sein.

Bevorzugt ist Ar Phenyl oder durch $C_1$-$C_4$-Alkyl, $C_1$-$C_4$-Alkoxy, Cl, Br oder $NO_2$ substituiertes Phenyl.

Das Verfahren eignet sich bevorzugt zur Herstellung von Verbindungen der Formel I, worin n = 1 ist.

Die sekundären Alkohole der Formel II sind bekannte Verbindungen und teilweise im Handel erhältlich.

Die zum Verfahren verwendeten Sulfonsäurechloride können aliphatische, cycloaliphatische oder aromatische Sulfonsäurechloride sein. Bevorzugt verwendet man aromatische Sulfochloride. R als Alkyl kann unverzweigt oder verzweigt sein und kann z.B. Methyl, Ethyl, Isopropyl, n-Butyl, sec. Butyl, tert. Butyl, Octyl, Dodecyl oder Octadecyl sein. R als aromatischer Rest kann z.B. Phenyl, Naphthyl, 4-Tolyl, 2-Tolyl,

2,4-Xylyl, 4-Isopropylphenyl, 4-Dodecylphenyl, 4-Tetradecylphenyl, Butylnaphthyl, Dodecylnaphthyl, 4-Fluorphenyl, 4-Chlorphenyl, 3,4-Dichlorphenyl, 4-Bromphenyl, 4-Methoxyphenyl, 4-Butoxyphenyl, 4-Biphenylyl, 3-Nitrophenyl oder 4-Chlor-3-nitrophenyl sein. Solche Sulfonsäurechloride sind bekannte Verbindungen und können z.B. durch Chlorsulfonierung der entsprechenden Grundkörper mit Sulfurylchlorid oder mit Chlorsulfonsäure gewonnen werden. Bevorzugt verwendet man ein Sulfonsäurechlorid der Formel $RSO_2Cl$, worin R Methyl, Phenyl oder Naphthyl oder durch Halogen oder $C_1$-$C_{14}$-Alkyl substituiertes Phenyl ist, insbesondere Toluolsulfochlorid oder Benzolsulfochlorid.

Beispiele für verwendbare Basen sind Alkali- oder Erdalkalimetallhydroxide, wie z.B. NaOH, KOH, LiOH, $Mg(OH)_2$ oder $Ba(OH)_2$; Erdalkalimetalloxide, wie CaO, MgO, SrO und Alkali- oder Erdalkalimetallalkoholate, wie z.B. $NaOCH_3$, $NaOC_2H_5$, $KOC_4H_9$-tert., $LiOC_5H_{11}$-tert., $NaOCH_2C_4H_9$-tert., $NaOC_6H_{13}$-n oder $Mg(OC_2H_5)_2$.

Pro Mol des sekundären Alkohols II verwendet man mindestens 1 Mol des Sulfochlorides $RSO_2Cl$, vorzugsweise verwendet man 1,0 bis 1,5 Mol $RSO_2Cl$.

Pro Mol des sekundären Alkohols II verwendet man mindestens 2 Aequivalente der Base, vorzugsweise 2 bis 3 Aequivalente.

Die Reaktion kann - bei Verwendung von NaOH als Base - durch folgende Gleichung wiedergegeben werden:

$$Ar{-}[CO]_n{-}CH(OH){-}Ar + RSO_2Cl + 2\ NaOH \longrightarrow$$

$$Ar{-}[CO]_n{-}CO{-}Ar + RSO_2Na + NaCl + 2\ H_2O$$

Als organische Lösungsmittel können beispielsweise Kohlenwasserstoffe, Ether, cyclische Ether, Ketone, Ester, Halogenkohlenwasserstoffe, Dimethylformamid, Dimethylsulfoxid, Tetramethylensulfon, Hexamethylphosphorsäuretriamid, N,N'-Dimethyl-ethylenharnstoff oder Dimethylacetamid verwendet werden. Beispiele für Kohlenwasserstoffe sind Benzol, Toluol, Xylol oder Cyclohexan; Beispiele für Ether und cyclische Ether sind Diethylether, Di-isopropylether, Dibutylether, Tetrahydrofuran oder Dioxan; Beispiele für Ketone sind Aceton, Methyl-ethylketon oder Methyl-isopropylketon; Beispiele für Ester sind Ethylacetat oder Butylacetat; Beispiele für Halogenkohlenwasserstoffe sind Tetrachlormethan, Methylenchlorid, Trichlorethylen, Tetrachlorethan oder Chlorbenzol.

Das Verfahren kann in An- oder Abwesenheit von Wasser ausgeführt werden. Die Anwesenheit von Wasser ist in solchen Fällen von Vorteil, wenn eine wasserlösliche Base verwendet wird. Bei Verwendung von Alkoholaten als Base empfiehlt sich die wasserfreie Durchführung des Verfahrens. Arbeitet man in Gegenwart von Wasser und einem mit Wasser nicht mischbaren Lösungsmittel, so empfiehlt sich der Zusatz eines Phasentransferkatalysators, wie z.B. eines Ammoniumsalzes, eines Kronenethers, eines Kryptates, eines Polyethylenglykols oder dessen Derivates oder eines Phosphoniumsalzes. Solche Katalysatoren werden zweckmässig in Mengen von 0,01 bis 10 Mol %, bezogen auf Verbindung II zugesetzt. Solche Phasentransferkatalysatoren beschleunigen die Reaktion und verkürzen somit die Reaktionszeit.

Die Reaktion kann ohne Erwärmen und ohne Kühlung durchgeführt werden, zur Verkürzung der Reaktionszeit empfiehlt sich jedoch ein Erwärmen bis maximal 150° C, vorzugsweise bis maximal 80° C.

Zur Isolierung der Produkte wird das Reaktionsgemisch vorzugsweise in eine wässrige und eine organische Phase überführt. In der organischen Phase befindet sich das Keton der Formel I und kann daraus z.B. durch Eindampfen und/oder Kristallisation gewonnen werden.

In der wässrigen Phase befindet sich das Basensalz der Sulfinsäure. Dieses kann durch Aussalzen oder durch Konzentration der wässrigen Lösung als solches isoliert werden oder man säuert die wässrige Lösung mit einer Mineralsäure an und isoliert die freie Sulfinsäure durch Filtration oder Extraktion mit einem organische Lösungsmittel. Für Routineversuche kann man den Gehalt an Sulfinsäure in der wässrigen Phase durch Titration mit einer standardisierten Alkalinitritlösung ermitteln, ohne die Sulfinsäure zu isolieren.

Diese Bestimmungsmethode beruht auf der allgemeinen Reaktion von Sulfinsäuren mit salpetriger Säure nach der Gleichung

$$2\ R-SO_2H + HNO_2 \longrightarrow R-SO_2-\overset{OH}{\overset{|}{N}}-SO_2-R + H_2O$$

Die überschüssige salpetrige Säure wird nach üblichen Methoden bestimmt, z.B. durch Titration mit saurer Nitroanilinlösung.

Sowohl die Sulfinsäuren als auch die Ketone fallen bei diesem Verfahren in einer Reinheit an, die für viele Verwendungszwecke genügend ist. Eine Reinigung der isolierten Ketone kann nach den üblichen Methoden wie z.B. Kristallisation oder Vakuumdestillation erfolgen. Isolierung und Reinigung der freien Sulfinsäuren muss mit entsprechender Sorgfalt geschehen, da sich diese Verbindungen leicht zersetzen und an der Luft zu den entsprechenden Sulfonsäuren oxidiert werden.

Aus letzterem Grund ist auch eine Recyclisierung der Sulfinsäuren zu den Sulfochloriden möglich, indem man die Sulfinsäure zur entsprechenden Sulfonsäure oxidiert und diese durch Reaktion mit einem Chlorierungsmittel (z.B. $PCl_5$) in das Sulfochlorid überführt.

Die nachstehenden Beispiele erläutern die Ausführung des Verfahrens näher, ohne das Verfahren auf diese Beispiele beschränken zu wollen.

Beispiel 1: Herstellung von Benzil und p-Toluolsulfinsäure

In ein Gemisch von 40 g (0,5 Mol) einer 50%igen wässrigen NaOH-Lösung und 200 ml Toluol werden unter Rühren 21.2 g (0.1 Mol) Benzoin eingetragen. Zu der entstandenen violetten Suspension werden 1.7 g Tetrabutylammoniumhydrogensulfat als Phasentransferkatalysator zugesetzt, wobei die Farbe auf grün wechselt. Die Suspension wird auf 50 °C erwärmt und bei dieser Temperatur unter schnellem Rühren innerhalb von 30-40 min mit einer Lösung von 20 g (0.105 Mol) p-Toluolsulfochlorid in 40 ml Toluol versetzt, wobei eine gelbe Emulsion entsteht. Anschliessend wird 1 h bei 50 °C nachgerührt.

Nach Abkühlen auf Raumtemperatur werden 200 ml Wasser zugegeben, wobei sich die zwei Phasen klar trennen. Die organische Phase wird über $MgSO_4$ getrocknet und im Vakuum eingedampft. Als Rückstand verbleiben 20,5 g Benzil, welches gemäss chromatographischer Analyse eine Reinheit von 99 % aufweist. Dies entspricht einer Ausbeute von 95 % der Theorie.

Eine Probe der wässrigen Phase wird zur Bestimmung der darin befindlichen Toluolsulfinsäure mit HCl angesäuert und mit einem Ueberschuss an 0.1 N $NaNO_2$-Lösung versetzt. In der entstandenen weissen Suspension wird die überschüssige $HNO_2$ mit 0.1 N Nitroanilinlösung zurücktitriert, bis ein bleibender Farbumschlag auf gelb erfolgt ist. Aus der Analyse ergibt sich ein Gehalt der wässrigen Phase an 16,6 g p-Toluolsulfinsäure-Natrium, was einer Ausbeute von 93 % der Theorie entspricht.

Zur Isolierung des Sulfinsäuresalzes wird die wässrige Lösung mit NaCl gesättigt. Es scheidet sich ein Niederschlag ab, der nach einstündigem Stehen abfiltriert wird. Nach dem Trocknen im Exsiccator erhält man 16,2 g rohes p-Toluolsulfinsäure-Natriumsalz, das noch NaCl enthält.

Zur Isolierung der freien p-Toluolsulfinsäure wird eine wässrige Lösung des Natriumsalzes mit Salzsäure angesäuert. Der ausgefallene Niederschlag wird abfiltriert und schmilzt nach kurzer Trocknung im Exsiccator bei 83 - 85 °C (Lit. Merck-Index Smp. 85 °C). Dasselbe Produkt erhält man auch durch Extraktion der sauren Lösung mit Diethylether und Eindampfen der Etherlösung.

Zum Strukturbeweis werden 4 g der rohen Sulfinsäure in 100 ml 1 N Salzsäure suspendiert und mit 0,9 g $NaNO_2$ versetzt. Der gebildete Niederschlag wird abfiltriert und im Exsiccator getrocknet. Man erhält 4 g N,N-Di-p-toluolsulfonyl-hydroxylamin, das nach Umkristallisation aus Ethanol bei 125 - 126 °C schmilzt (Lit. Beilstein XI, 109, Smp. 125 °C).

Beispiele 2 - 9

Analog zu Beispiel 1 werden jeweils 0.1 Mol sekundärer Alkohol mit 0,105 Mol Sulfochlorid und 0,5 Aequivalenten Base unter Variation der Ausgangskomponenten und des Lösungsmittels umgesetzt. Die in der folgenden Tabelle 1 angegebene Ausbeute an Keton entspricht dem Eindampfrest der organischen Phase, die Reinheit des Ketons wird durch Chromatographie bestimmt. Die angegebene Ausbeute an Sulfinsäure wird durch Analyse der wässrigen Phase (wie in Beispiel 1 beschrieben) ermittelt. Als Phasentransferkatalysator wird in den Beispielen 6 und 7 Tetrabutylammoniumbromid, in allen anderen Beispielen Tetrabutylammonium-hydrogensulfat verwendet.

**Tabelle 1:**

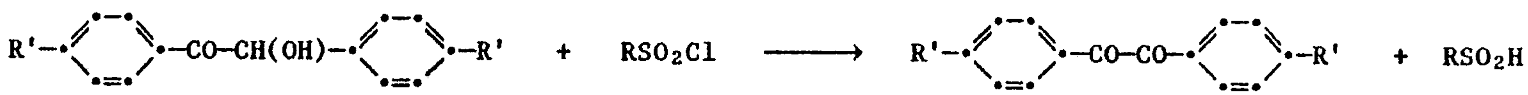

| Beispiel Nr. | R' | R | Lösungsmittel | Base | Ausbeute/Reinheit Keton | Ausbeute Sulfinsäure |
|---|---|---|---|---|---|---|
| 2 | H | $C_8H_{17}$ | 230 ml Toluol | NaOH | 98 % / 87 % | 53 % |
| 3 | H | 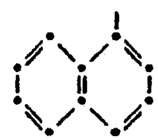 | 240 ml Toluol | NaOH | 97 % / 92 % | 81 % |
| 4 | H | " | 200 ml Chlorbenzol | NaOH | 99 % / 90 % | 69 % |
| 5 | H | 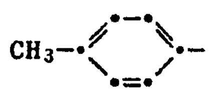 | 230 ml Toluol | KOH | 92 % / 99 % | 79 % |

Tabelle 1: (Fortsetzung)

| Beispiel Nr. | R' | R | Lösungsmittel | Base | Ausbeute/Reinheit Keton | Ausbeute Sulfinsäure |
|---|---|---|---|---|---|---|
| 6 | H | 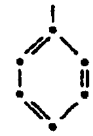 | 230 ml Toluol | NaOH | 98 % / 98 % | 66 % |
| 7 | $CH_3O$ | 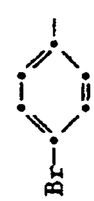 | 440 ml Toluol | NaOH | 95 % / 96 % | 65 % |
| 8 | $CH_3O$ | 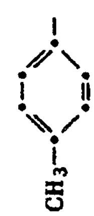 | 390 ml Toluol | NaOH | 95 % / 95 % | 58 % |
| 9 | H | 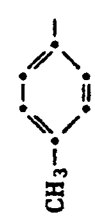 | 240 ml Toluol | $Ba(OH)_2$ | 82 % / 91 % | 85 % |

Beispiel 10: Herstellung von Benzophenon

Eine Lösung von 18.4 g (0.1 Mol) Benzhydrol in 160 ml Tetrahydrofuran wird mit 24,7 g (0.22 Mol) Kalium-tert-butylat versetzt und unter Rühren auf 50° C erwärmt. Bei dieser Temperatur wird eine Lösung

von 21 g (0,12 Mol) p-Toluolsulfochlorid in 40 ml Tetrahydrofuran in 45 - 60 min zugetropft. Die entstandene weisse Suspension wird noch 1 h nachgerührt, dann auf Raumtempeatur gekühlt und mit 200 ml Wasser und 200 ml Toluol versetzt.

Die organische Phase wird über $MgSO_4$ getrocknet und im Vakuum eingedampft. Als Rückstand verbleiben 17.8 g rohes Benzophenon, welches eine Reinheit von 80 % aufweist.

Beispiele 11 - 13

Analog zu Beispiel 10 werden jeweils 0,1 Mol Carbinol in Gegenwart von 0,22 Mol Kalium-tert-butylat in Tetrahydrofuran (THF) umgesetzt. Die Ergebnisse sind in Tabelle 2 aufgeführt.

**Tabelle 2:**

| Beispiel Nr. | Verwendetes Carbinol | Menge THF | Sulfochlorid | Ausbeute/Reinheit Keton | Ausbeute Sulfinsäure als K-Salz |
|---|---|---|---|---|---|
| 11 | Benzoin | 240 ml | 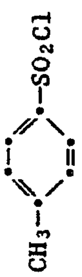 0,105 Mol | 97 % / 98 % | 85 % |
| 12 | Benzoin | 230 ml | $CH_3SO_2Cl$ 0,105 Mol | 96 % / 97 % | 57 % |
| 13 | α-Pyridoin | 440 ml | 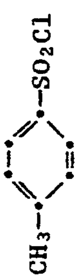 0,11 Mol | 96 % / 95 % | 67 % |

## Ansprüche

**1.** Verfahren zur Herstellung eines aromatischen Ketons der Formel I

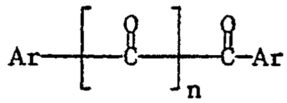

(I)

worin Ar ein aromatischer carbocyclischer Rest mit 6-14 C-Atomen oder aromatischer heterocyclischer Rest mit 4 - 14 C-Atomen ist, der unsubstituiert oder durch einen oder mehrere der Reste Halogen, $C_1$-$C_{14}$-Alkyl, $C_1$-$C_8$-Alkoxy, Phenyl oder Nitro substituiert sein kann, und n 0 oder 1 ist, unter gleichzeitiger Bildung einer Sulfinsäure der Formel R-S(O)-OH oder deren Alkalimetall- oder Erdalkalimetallsalz, worin R $C_1$-$C_{18}$-Alkyl, 10-Campheryl, Phenyl, Naphthyl oder durch Halogen, $C_1$-$C_{14}$-Alkyl, $C_1$-$C_{12}$-Alkoxy oder Phenoxy substituiertes Phenyl oder Naphthyl ist, dadurch gekennzeichnet, dass man einen sekundären Alkohol der Formel II

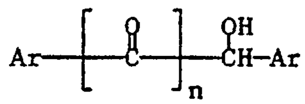

(II)

mit einem Sulfonsäurechlorid der Formel R-$SO_2$-Cl in Gegenwart einer Base, ausgewählt aus Alkali- oder Erdalkalimetall-hydroxiden, -oxiden oder -alkoholaten, und in Gegenwart eines organischen Lösungsmittels bei einer Temperatur vcn 20° - 150°C umsetzt, wobei pro Mol des Alkohols II mindestens 1 Mol $RSO_2Cl$ und mindestens 2 Aequivalente der Base verwendet werden.

2. Verfahren gemäss Anspruch 1, dadurch gekennzeichnet, dass die Reaktion bei 20 - 80°C ausgeführt wird.

3. Verfahren gemäss Anspruch 1, dadurch gekennzeichnet, dass die Reaktion wasserfrei in einem organischen Lösungsmittel ausgeführt wird und als Base ein Alkalimetallalkoholat verwendet wird.

4. Verfahren gemäss Anspruch 1, dadurch gekennzeichnet, dass die Reaktion in heterogener Phase ausgeführt wird und als Base eine wässrige Lösung eines Alkalimetallhydroxides verwendet wird.

5. Verfahren gemäss Anspruch 4, dadurch gekennzeichnet, dass dem Reaktionsgemisch ein Phasentransferkatalysator zugesetzt wird.

6. Verfahren gemäss Anspruch 1, dadurch gekennzeichnet, dass ein Sulfonsäurechlorid $RSO_2Cl$ verwendet wird, worin R Methyl, Phenyl oder Naphthyl oder durch Halogen oder $C_1$-$C_{14}$-Alkyl substituiertes Phenyl ist.

7. Verfahren gemäss Anspruch 6, dadurch gekennzeichnet, dass Toluolsulfochlorid oder Benzolsulfochlorid verwendet wird.

8. Verfahren gemäss Anspruch 1 zur Herstellung von Ketonen der Formel I aus Alkoholen der Formel II, dadurch gekennzeichnet, dass in Formel I und II Ar Phenyl oder durch $C_1$-$C_4$-Alkyl, $C_1$-$C_4$-Alkoxy, Cl, Br oder $NO_2$ substituiertes Phenyl ist.

9. Verfahren gemäss Anspruch 8, dadurch gekennzeichnet, dass in Formel I und II Ar Phenyl ist.

10. Verfahren gemäss Anspruch 1 zur Herstellung von Ketonen der Formel I aus Alkoholen der Formel II, dadurch gekennzeichnet, dass in Formel I und II n = 1 ist.

## Claims

1. A process for the preparation of an aromatic ketone of formula I

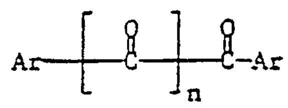

(I)

in which Ar is an aromatic carbocyclic radical having 6 - 14 C atoms or aromatic heterocyclic radical having 4 - 14 C atoms, which may be unsubstituted or substituted by one or more radicals of the group consisting of halogen, $C_1$-$C_{14}$alkyl, $C_1$-$C_8$alkoxy, phenyl or nitro, and n is 0 or 1, with simultaneous formation of a sulfinic acid of formula R-S(O)-OH, or the alkali metal salt or alkaline-earth metal salt thereof, in which R is $C_1$-$C_{18}$alkyl, 10-camphoryl, phenyl, naphthyl, or phenyl or naphthyl which are substituted by halogen, $C_1$-$C_{14}$alkyl, $C_1$-$C_{12}$alkoxy or phenoxy, which comprises reacting a secondary alcohol of formula II

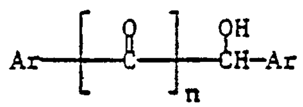

(II)

with a sulfonyl chloride of formula R-$SO_2$-Cl, in the presence of a base selected from the group consisting of hydroxides, oxides and alcoholates of alkali metals or alkaline-earth metals, and in the presence of an organic solvent, at a temperature from 20 – 150 °C, such that not less than 1 mole of $RSO_2Cl$ and not less than 2 equivalents of the base are used per mole of the alcohol II.

2. A process according to claim 1, wherein the reaction is carried out at 20 - 80 °C.

3. A process according to claim 1, wherein the reaction is carried out under anhydrous conditions in an organic solvent, using an alkali metal alcoholate as base.

4. A process according to claim 1, wherein the reaction is carried out in heterogeneous phase, using an aqueous solution of an alkali metal hydroxide as base.

5. A process according to claim 4, wherein a phase transfer catalyst is added to the reaction mixture.

6. A process according to claim 1, wherein a sulfonyl chloride of formula $RSO_2Cl$ is used, in which R is methyl, phenyl or naphthyl, or phenyl which is substituted by halogen or $C_1$-$C_{14}$alkyl.

7. A process according to claim 6, wherein toluenesulfonyl chloride or benzenesulfonyl chloride is used.

8. A process according to claim 1 for the preparation of a ketone of formula I from an alcohol of formula II, wherein in formulae I and II Ar is phenyl, or phenyl which is substituted by $C_1$-$C_4$alkyl, $C_1$-$C_4$alkoxy, Cl, Br or $NO_2$.

9. A process according to claim 8, wherein in formulae I and II Ar is phenyl.

10. A process according to claim 1 for the preparation of a ketone of formula I from an alcohol of formula II, wherein in formulae I and II n is 1.

## Revendications

1. Procédé pour préparer une cétone aromatique répondant à la formule I

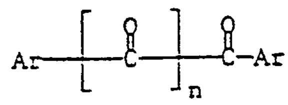

(I)

dans laquelle Ar représente un radical carbocyclique aromatique contenant de 6 à 14 atomes de carbone ou un radical hétérocyclique aromatique contenant de 4 à 14 atomes de carbone, non substitué ou porteur d'un ou de plusieurs substituants pris dans l'ensemble constitué par les halogènes, les alkyles en $C_1$-$C_{14}$, les alcoxy en $C_1$-$C_8$, le phényle et le nitro, et n est égal à 0 ou à 1, avec formation simultanée d'un acide sulfinique répondant à la formule :

R-S(O)-OH

dans laquelle R représente un alkyle en $C_1$-$C_{18}$, un camphéryle-10, un phényle, un naphtyle ou un radical phényle ou naphtyle porteur d'un halogène, d'un alkyle en $C_7$-$C_{14}$, d'un alcoxy en $C_1$-$C_{12}$ ou d'un phénoxy,
ou d'un sel de métal alcalin ou de métal alcalino-terreux d'un tel acide sulfinique, procédé caractérisé en ce qu'on fait réagir à une température de 20 à 150 °C un alcool secondaire de formule II

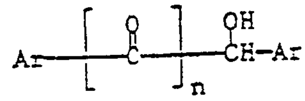

(II)

avec un chlorure d'acide sulfonique de formule R-$SO_2$-Cl, en présence d'une base prise dans l'ensemble constitué par les hydroxydes, les oxydes et les alcoolates des métaux alcalins et des métaux alcalino-terreux, et en rpésence d'un solvant organique, en utilisant, par mole de l'alcool II, au moins 1 mol de $RSO_2Cl$ et au moins 2 équivalents de la base.

2. Procédé selon la revendication 1 caractérisé en ce que la réaction est exécutée à une température de 20 à 80 °C.

3. Procédé selon la revendication 1 caractérisé en ce que la réaction est effectuée à l'abri de l'eau dans un solvant organique et en ce qu'on utilise, comme base, un alcoolate de métal alcalin.

4. Procédé selon la revendication 1 caractérisé en ce que la réaction est effectuée en phase hétérogène et en ce qu'on utilise, comme base, une solution aqueuse d'un hydroxyde de métal alcalin.

5. Procédé selon la revendication 4 caractérisé en ce qu'on ajoute au mélange réactionnel un catalyseur de transfert de phase.

6. Procédé selon la revendication 1 caractérisé en ce qu'on utilise un chlorure d'acide sulfonique $RSO_2Cl$ dans lequel R représente un radical méthyle, phényle ou naphtyle ou un radical phényle porteur d'un halogène ou d'un alkyle en $C_1$-$C_{14}$.

7. Procédé selon la revendication 6 caractérisé en ce qu'on utilise un chlorure de toluène-sulfonyle ou le chlorure de benzène-sulfonyle.

8. Procédé selon la revendication 1 pour la préparation de cétones de formule I à partir d'alcools de formule II, procédé caractérisé en ce que, dans les formules I et II, Ar représente un phényle ou un phényle porteur d'un alkyle en $C_1$-$C_4$, d'un alcoxy en $C_1$-$C_4$, d'un atome de chlore, d'un atome de brome ou d'un radical $NO_2$.

9. Procédé selon la revendication 8 caractérisé en ce que, dans les formules I et II, Ar représente un radical phényle.

10. Procédé selon la revendication 1 pour la préparation de cétones de formule I à partir d'alcools de formule II, procédé caractérisé en ce que, dans les formules I et II, n est égal à 1.